# EUROPEAN PATENT APPLICATION

(11) **EP 4 421 095 A1**
(43) Date of publication of application: **28.08.2024**
(21) Application number: 22882743.2
(22) Date of filing: 13.10.2022
(51) Int. Cl.: C07K 16/30, C12N 15/11, C12N 15/63, C12N 5/07, C07K 19/00, A61K 39/395, A61P 35/00, G01N 33/577

(54) **ANTI-MESOTHELIN NANOBODIES AND USE THEREOF**

(30) Priority: 18.10.2021 CN 202111208871
(71) Applicant: Biotheus Inc., Tangjiawan Town, Xiangzhou District Zhuhai, Guangdong 519080 (CN)
(72) Inventor: ZHANG, Zhenqing, Zhuhai, Guangdong 519080 (CN); MIAO, Xiaoniu, Zhuhai, Guangdong 519080 (CN); WU, Fan, Zhuhai, Guangdong 519080 (CN); LI, Zhiyuan, Zhuhai, Guangdong 519080 (CN)
(74) Representative: Casci, Tamara
(86) International application number: PCT/CN2022/125135
(87) International publication number: WO 2023/066133

(57) **Abstract**

The present invention relates to nanobodies that specifically bind to MSLN or antigen-binding fragments thereof, a composition containing the nanobodies or antigen-binding fragments thereof, a nucleic acid encoding the antibodies or antigen-binding fragments thereof and a host cell comprising same, and a related use. In addition, the invention relates to therapeutic and diagnostic uses of these antibodies or antigen-binding fragments thereof.

## Description

### Technical Field

The present application relates to a nanobody or antigen-binding fragment thereof that specifically binds to MSLN, a composition comprising the nanobody or antigen-binding fragment thereof, a nucleic acid encoding the antibody or antigen-binding fragment thereof and a host cell comprising the same, and related uses thereof. Furthermore, the present invention relates to a therapeutic and diagnostic use of the antibody or antigen-binding fragment thereof.

### Background Art

Mesothelin (MSLN) is a 40 kDa cell surface glycoprotein that is highly expressed in pancreatic cancer, ovarian cancer, mesothelioma and some other cancers. Since MSLN can also be present in the blood of a small number of patients in secreted soluble form of sMSLN, measurement of MSLN in the blood may be useful in diagnosing and tracking patients' conditions. The MSLN gene encodes a precursor protein with a molecular weight of 69 kDa, which is processed into a 40 kDa membrane-bound protein (called MSLN) and a 31 kDa shed fragment called megakaryocyte-potentiating factor (MPF), and the fragment is secreted from the cell. MSLN is not a cancer-specific antigen and can be expressed in mesothelial cells of normal pleura, pericardium and peritoneum, but it is highly expressed in a variety of cancer cells. The limited distribution of MSLN on normal tissues makes it a promising target for tumor-specific therapy.

Currently, a variety of antibody drugs or cell therapies targeting MSLN have advanced to the clinical stage. For example, the antibody-conjugated drug Anetumab ravtansine developed by Bayer, ImmunoGen, Morphosys and other companies has been used for treatment of solid tumors in phase II clinical trials (NCT03926143); Amatuximab, a monoclonal antibody drug targeting MSLN developed by Morphotek, has also been used for clinical treatment of solid tumors and currently in phase II clinical trials; in addition, the Chinese People's Liberation Army Military Medical College and TCR² Therapeutics have also developed CAR-T therapies targeting MSLN, which are all currently in the clinical trial stage. Existing clinical trials mostly show that mesothelin-targeting therapies are safe, but the therapeutic effects are modest.

Single domain antibody (sdAb), also known as nanobody or heavy chain antibody (hcAb), is an antibody isolated from the sera of camelids and sharks, and its volume is about 1/10 of that of traditional antibodies. Different from traditional antibodies, single-domain antibody is only composed of heavy chain, and its antigen-binding region is only a single domain connected to an Fc region through a hinge region, and this antigen-binding region still has the ability to bind an antigen after being separated from the antibody. Single domain antibodies have the characteristics of small molecular weight and good stability. Compared with traditional normal antibodies in drug development and diagnostic reagent development, they have good tissue infiltration, flexible administration, high degree of humanization, easy transformation into recombinant protein and many other advantages.

### Contents of the present application

The inventors of the present application have screened and obtained an anti-MSLN nanobody after extensive research. The nanobody has high binding activity to MSLN and has cross-reactivity with human, monkey and/or mouse MSLN. In addition, the nanobody also has the characteristics of small molecular weight and good stability. Compared with traditional and common antibodies in drug development and diagnostic reagent development, it has good tissue infiltration, flexible administration, high degree of humanization, easy transformation into recombinant protein and many other advantages.

Based on this, the present application also provides a composition comprising the nanobody or antigen-binding fragment thereof, a nucleic acid encoding the nanobody or antigen-binding fragment thereof, a host cell comprising the same, and related uses thereof.

### Nanobody and antigen-binding fragment thereof

Therefore, in a first aspect, the present application provides a nanobody or antigen-binding fragment thereof capable of specifically binding to MSLN. The nanobody described herein is usually composed of 4 framework regions (FRs) and 3 complementarity-determining regions (CDRs), called FR1, CDR1, FR2, CDR2, FR3, CDR3 and FR4. The antigen-binding fragment comprises at least a portion of the nanobody that is sufficient to confer the ability of the fragment to specifically bind MSLN. In some embodiments, the nanobody of the present application can be truncated at the N- or C-terminus so that it comprises only part of FR1 and/or FR4, or lacks one or two of those framework regions, as long as it substantially maintains the ability and specificity to bind the antigen.

In certain embodiments, the nanobody or antigen-binding fragment thereof comprises:
a CDR1 or variant thereof, a CDR2 or variant thereof, a CDR3 or variant thereof contained in the variable region (VHH) as set forth in any one of SEQ ID NOs: 4 and 6-9;
wherein, the variant has a substitution, deletion or addition of one or several amino acids (e.g., a substitution, deletion or addition of 1, 2 or 3 amino acids) as compared to the sequence from which it is derived. In certain embodiments, the substitution is a conservative substitution.

In certain embodiments, the CDRs are defined according to the IMGT, Kabat or Chothia numbering system.

In certain embodiments, the nanobody or antigen-binding fragment thereof comprises:
a CDR1 having the sequence as set forth in SEQ ID NO: 1 or variant thereof, a CDR2 having the sequence as set forth in SEQ ID NO: 2 or variant thereof, and a CDR3 having the sequence as set forth in SEQ ID NO: 3 or variant thereof;
wherein, the variant has a substitution, deletion or addition of one or several amino acids (e.g., a substitution, deletion or addition of 1, 2 or 3 amino acids) as compared to the sequence from which it is derived. In certain embodiments, the substitution is a conservative substitution.

In certain embodiments, the nanobody or antigen-binding fragment thereof comprises: a CDR1 having the sequence as set forth in SEQ ID NO: 1, a CDR2 having the sequence as set forth in SEQ ID NO: 2, and a CDR3 having the sequence as set forth in SEQ ID NO: 3.

In certain embodiments, the CDRs are defined by the IMGT numbering system.

In certain embodiments, the nanobody or antigen-binding fragment thereof comprises an amino acid sequence selected from:
(i) the sequence as set forth in SEQ ID NO: 4;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 4; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 4.

In certain embodiments, the substitution is a conservative substitution.

In certain embodiments, the nanobody or antigen-binding fragment thereof is humanized.

In certain embodiments, the nanobody or antigen-binding fragment thereof further comprises a heavy chain framework region of a human immunoglobulin (e.g., a heavy chain framework region contained in the amino acid sequence encoded by a human immunoglobulin heavy chain germline gene), and the heavy chain framework region optionally comprises one or more (e.g., 1, 2, 3, 4, 5, 6, 7, 8, 9 or 10) back mutations from human residues to camel residues.

In certain embodiments, the nanobody or antigen-binding fragment thereof comprises:
(i) an FR1 as set forth in SEQ ID NO: 10, or having a substitution, deletion or addition of one or several amino acids (e.g., a substitution, deletion or addition of 1, 2, 3, 4 or 5 amino acids) as compared to SEQ ID NO: 10, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to SEQ ID NO: 10;
(ii) an FR2 as set forth in any one of SEQ ID NOs: 11-13, or having a substitution, deletion or addition of one or several amino acids (e.g., a substitution, deletion or addition of 1, 2, 3, 4 or 5 amino acids) as compared to any one of SEQ ID NOs: 11-13, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to any one of SEQ ID NOs: 11-13;
(iii) an FR3 as set forth in any one of SEQ ID NOs: 14-16, or having a substitution, deletion or addition of one or several amino acids (e.g., a substitution, deletion or addition of 1, 2, 3, 4 or 5 amino acids) as compared to any one of SEQ ID NOs: 14-16, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to any one of SEQ ID NOs: 14-16;
   and/or,
(iv) an FR4 as set forth in SEQ ID NO: 17, or having a substitution, deletion or addition of one or several amino acids (e.g., a substitution, deletion or addition of 1, 2, 3, 4 or 5 amino acids) as compared to SEQ ID NO: 17, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to SEQ ID NO: 17.

In certain embodiments, the nanobody or antigen-binding fragment thereof comprises an amino acid sequence selected from:
(i) the sequence as set forth in any one of SEQ ID NOs: 6-9;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 6-9; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 6-9.

In certain embodiments, the substitution is a conservative substitution.

### Polypeptide construct

In a second aspect, the present application also provides a polypeptide construct capable of specifically binding MSLN, which comprises the nanobody or antigen-binding fragment thereof as described above, and an immunoglobulin Fc domain.

In this context, the Fc domain, also called Fc region, refers to a part of heavy chain constant region that contains CH2 and CH3 domains. In some embodiments, the Fc domain comprises a hinge, a CH2 domain, and a CH3 domain. When the Fc domain comprises a hinge, the hinge mediates dimerization between two Fc-containing polypeptides. The Fc domain can be of any antibody heavy chain constant region isotype. In some embodiments, the Fc domain is an Fc domain of IgGl, IgG2, IgG3 or IgG4.

In certain embodiments, the Fc domain comprised by the polypeptide construct of the present application is a native Fc region, which comprises an amino acid sequence consistent with the amino acid sequence of an Fc region found in nature. For example, the Fc domain may be a human IgG1 Fc region with a native sequence, a human IgG2 Fc region with a native sequence, a human IgG3 Fc region with a native sequence, or a human IgG4 Fc region with a native sequence. Native Fc regions can have effector functions. Exemplary "effector functions" include binding to Fc receptors; Clq binding and complement-dependent cytotoxicity (CDC); antibody-dependent cell-mediated cytotoxicity (ADCC); phagocytosis; downregulation of cell surface receptors (e.g., B cell receptors); and B cell activation, etc. Functional changes can be produced by replacing at least one amino acid residue in the native Fc region with a different residue or by chemical modification, and such changes include, for example, changing the affinity of antibody for an effector ligand (e.g., FcR or complement component C1q), thereby altering (e.g., lowering or boosting) effector functions.

Therefore, in certain embodiments, the Fc domain comprised by the polypeptide construct of the present application may also be a variant Fc region, which may comprise one or more (e.g., 1 to 10, such as 1 to 5) amino acid mutations or chemical modifications as compared to the native Fc region to change one or more of the following properties of the antibody of the present application: Fc receptor binding, antibody glycosylation, number of cysteine residues, effector cell function or complement function, etc..

In certain embodiments, the Fc domain comprised by the polypeptide construct of the present application possesses ADCC activity. In certain embodiments, the Fc domain comprised by the polypeptide construct of the present application does not possess ADCC activity.

In certain embodiments, the immunoglobulin Fc domain is linked to the N-terminus and/or C-terminus (e.g., C-terminus) of the nanobody or antigen-binding fragment thereof optionally via a peptide linker.

In certain embodiments, the immunoglobulin Fc domain is an IgG Fc domain (e.g., an IgGl Fc domain).

In certain embodiments, the immunoglobulin Fc domain comprises the sequence as set forth in SEQ ID NO: 5, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion or addition of one or more amino acids (e.g., a substitution, deletion or addition of 1, 2, 3, 4 or 5 amino acids) as compared thereto.

### Preparation of nanobody and polypeptide construct

The nanobody or polypeptide construct of the present application can be prepared by various methods known in the art, for example, obtained by genetic engineering recombinant technology. For example, a DNA molecule encoding the nanobody or polypeptide construct of the present application can be obtained through chemical synthesis or PCR amplification. The resulting DNA molecule is inserted into an expression vector and then transfected into a host cell. Then, the transfected host cell is cultured under a specific condition and expresses the antibody or polypeptide construct of the present application.

The antigen-binding fragments of the present application can be obtained by hydrolysis of an intact nanobody molecule (see, Morimoto et al., J. Biochem. Biophys. Methods 24:107-117 (1992) and Brennan et al., Science 229:81 (1985)). Besides, these antigen-binding fragments can also be produced directly from recombinant host cells (reviewed in Hudson, Curr. Opin. Immunol. 11: 548-557 (1999); Little et al., Immunol. Today, 21: 364-370 (2000)). Those of ordinary skill in the art are well aware of other techniques for preparing such antigen-binding fragments.

### Nucleic acid molecule

In a third aspect, the present application also provides an isolated nucleic acid molecule, which encodes the nanobody or antigen-binding fragment thereof as described above or the polypeptide construct as described above.

### Vector

In a fourth aspect, the present application also provides a vector, which comprises the nucleic acid molecule as described above. In certain embodiments, the vector is a cloning vector or an expression vector.

### Host cell

In a fifth aspect, the present application also provides a host cell, which comprises the nucleic acid molecule or vector as described above. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, such as mouse cell, human cell, etc.).

### Preparation method

In a sixth aspect, the present application also provides a method for preparing the nanobody or antigen-binding fragment thereof as described above or the polypeptide construct as described above, which comprises culturing the host cell as described above under a condition that allows protein expression, and recovering the nanobody or antigen-binding fragment thereof or the polypeptide construct from a culture of the cultured host cell.

### Bispecific or multispecific antibody

In a seventh aspect, the present application also provides a bispecific or multispecific antibody, which comprises the nanobody or antigen-binding fragment thereof as described above or the polypeptide constructs as described above. The present application also provides a use of the nanobody or antigen-binding fragment thereof or polypeptide construct of the present application, or nucleic acid molecule, vector or host cell encoding them in the manufacture of a bispecific or multispecific antibody.

In certain embodiments, the bispecific or multispecific antibody specifically binds MSLN and additionally specifically binds one or more other targets.

In certain embodiments, the bispecific or multispecific antibody further comprises at least one second antibody having a second binding specificity for a second target.

### Conjugate

In an eighth aspect, the present application also provides a conjugate, which comprises the nanobody or antigen-binding fragment thereof as describe above or the polypeptide construct as described above, and a therapeutic agent conjugated to the nanobody or antigen-binding fragment thereof or the polypeptide construct as described above. The present application also provides a use of the nanobody or antigen-binding fragment thereof or polypeptide construct of the present application, or nucleic acid molecule, vector or host cell encoding them in the manufacture of a conjugate.

In certain embodiments, the therapeutic agent is selected from the group consisting of antineoplastic agents, such as cytotoxic agent, hormonal agent, biological response modifier, additional antibody or antigen-binding fragment thereof.

### Chimeric antigen receptor

In a ninth aspect, the present application also provides a chimeric antigen receptor, which comprises an antigen-binding domain, a spacer domain, a transmembrane domain, and an intracellular signaling domain (e.g., a primary signaling domain and/or a costimulatory signaling domain), wherein the antigen-binding domain comprises the nanobody or antigen-binding fragment thereof as described above. The present application also provides a use of the nanobody or antigen-binding fragment thereof or polypeptide construct of the present application, or nucleic acid molecule, vector or host cell encoding them in the manufacture of a chimeric antigen receptor or an immune cell expressing the chimeric antigen receptor.

In certain embodiments, the antigen binding domain confers the ability of recognizing MSLN to the chimeric antigen receptor; the spacer domain facilitates structural flexibility of the protein and enables the movement of one or two domains in relation to each other; the transmembrane domain can be thermodynamically stable when associated with a cell membrane (especially an eukaryotic cell membrane); the intracellular signaling domain is involved in transmitting effective antigen receptor-binding signals into the interior of immune effector cell, activating at least one normal effector function of CAR-expressing immune effector cell, or enhancing the secretion of at least one cytokine by the CAR-expressing immune effector cell.

In certain embodiments, the chimeric antigen receptor is expressed by an immune effector cell (e.g., a T cell).

In a tenth aspect, the present application also provides an isolated nucleic acid molecule, which encodes the chimeric antigen receptor as described above.

In an eleventh aspect, the present application also provides a vector, which comprises an isolated nucleic acid molecule as described above. In certain embodiments, the isolated nucleic acid molecule is used to prepare a chimeric antigen receptor T cell.

In a twelfth aspect, the present application also provides a host cell, which comprises the isolated nucleic acid molecule or vector as described above.

In certain embodiments, the host cell is an immune effector cell (e.g., a T cell or a NK cell).

In certain embodiments, the host cell is a chimeric antigen receptor T cell (CAR-T).

### Pharmaceutical composition

In the thirteenth aspect, the present application also provides a pharmaceutical composition, which comprises the nanobody or antigen-binding fragment thereof of the first aspect, the polypeptide construct of the second aspect, the bispecific or multispecific antibody of the seventh aspect, the conjugate of the eighth aspect, the chimeric antigen receptor of the ninth aspect, the isolated nucleic acid molecule of the third or tenth aspect, the vector of the fourth or eleventh aspect, or the host cell of the fifth or twelfth aspect.

In certain embodiments, the pharmaceutical composition further comprises a pharmaceutically acceptable carrier and/or excipient.

In certain exemplary embodiments, the pharmaceutically acceptable carrier and/or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

In certain embodiments, the pharmaceutical composition comprises the nanobody or antigen-binding fragment thereof of the first aspect, the polypeptide construct of the second aspect, the isolated nucleic acid molecule of the third aspect, the vector of the fourth aspect, or the host cell of the fifth aspect.

In certain embodiments, the pharmaceutical composition comprises the bispecific or multispecific antibody of the seventh aspect as described above.

In certain embodiments, the pharmaceutical composition comprises the conjugate of the eighth aspect as described above.

In certain embodiments, the pharmaceutical composition comprises the chimeric antigen receptor of the ninth aspect, the isolated nucleic acid molecule of the tenth aspect, the vector of the eleventh aspect, or the host cell of the twelfth aspect as described above.

### Pharmaceutical use

In the fourteenth aspect, the present application also provides a use of the nanobody or antigen-binding fragment thereof of the first aspect, the polypeptide construct of the second aspect, the bispecific or multispecific antibody of the seventh aspect, the conjugate of the eighth aspect, the chimeric antigen receptor of the ninth aspect, the isolated nucleic acid molecule of the third or tenth aspect, the vector of the fourth or eleventh aspect, the host cell of the fifth or twelfth aspect, or the pharmaceutical composition of the thirteenth aspect, in the manufacture of a medicament for preventing and/or treating a tumor in a subject.

In certain embodiments, the tumor is a MSLN-positive tumor.

In certain embodiments, the tumor is selected from the group consisting of solid tumors, such as gastric cancer, lung cancer, ovarian cancer, esophageal cancer, pancreatic cancer, cervical cancer, mesothelioma, or breast cancer. It is known in the art that such tumors highly express MSLN (see, for example, Aurore et al., cancer discovery, 2016).

In certain embodiments, the subject is a mammal, such as a human.

In certain embodiments, the nanobody or antigen-binding fragment thereof, polypeptide construct, isolated nucleic acid molecule, vector, host cell, bispecific or multispecific antibody, conjugate, chimeric antigen receptor, or pharmaceutical composition is used alone or in combination with an additional pharmaceutically active agent (e.g., an antineoplastic agent).

### Method for prevention and/or treatment of tumors

In a fifteenth aspect, the present application also provides a method for preventing and/or treating a tumor in a subject, which comprises: administering to the subject in need thereof an effective amount of the nanobody or antigen-binding fragment thereof of the first aspect, the polypeptide construct of the second aspect, the bispecific or multispecific antibody of the seventh aspect, the conjugate of the eighth aspect, the chimeric antigen receptor of the ninth aspect, the isolated nucleic acid molecule of the third or tenth aspect, the vector of the fourth or eleventh aspect, the host cell of the fifth or twelfth aspect, or the pharmaceutical composition of the thirteenth aspect.

In certain embodiments, the tumor is a MSLN-positive tumor.

In certain embodiments, the tumor is selected from the group consisting of solid tumors, such as gastric cancer, lung cancer, ovarian cancer, esophageal cancer, pancreatic cancer, cervical cancer, mesothelioma, or breast cancer.

In certain embodiments, the subject is a mammal, such as a human.

The nanobody or antigen-binding fragment thereof, polypeptide construct, bispecific or multispecific antibody, or pharmaceutical composition of the present application can be formulated into any dosage form known in the medical field, such as tablets, pills, suspensions, emulsions, solutions, gels, capsules, powders, granules, elixirs, lozenges, suppositories, injections (including solutions for injection, sterile powders for injection and concentrated solutions for injection), inhalants, sprays, etc. The preferred dosage form depends on the intended mode of administration and therapeutic use. The nanobody or antigen-binding fragment thereof, polypeptide construct, bispecific or multispecific antibody, or pharmaceutical composition of the present application should be sterile and stable under production and storage conditions. One preferred dosage form is an injectable solution. Such injectable solution may be a sterile injectable solution. For example, a sterile injectable solution can be prepared by incorporating a necessary dosage of the nanobody or antigen-binding fragment thereof, polypeptide construct, bispecific or multispecific antibody, or pharmaceutical composition of the present application into an appropriate solvent, and optionally, simultaneously incorporating other desired ingredients (including, but not limited to, pH adjuster, surfactant, adjuvant, ionic strength enhancer, isotonic agent, preservative, diluent, or any thereof combination) thereto, followed by filter sterilization. Additionally, a sterile injectable solution may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

The nanobody or antigen-binding fragment thereof, polypeptide construct, bispecific or multispecific antibody, or pharmaceutical composition of the present application can be administered by any suitable method known in the art, including but not limited to, oral, buccal, sublingual, ophthalmic, local, parenteral, rectal, intrathecal, intra-cisternal, inguinal, intravesical, topical (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the nanobody or antigen-binding fragment thereof, polypeptide construct, bispecific or multispecific antibody, or pharmaceutical composition of the present application is administered by intravenous injection or bolus injection.

### Detection application

### Conjugate

In a sixteenth aspect, the present application also provides a conjugate, which comprises the nanobody or antigen-binding fragment thereof as described above or the polypeptide construct as described above, and a detectable label conjugated to the nanobody or antigen-binding fragment thereof or the polypeptide construct. In certain embodiments, the detectable label is, for example, an enzyme (e.g., horseradish peroxidase or alkaline phosphatase), a chemiluminescent reagent (e.g., an acridinium ester compound, luminol and derivative thereof, or ruthenium derivative), a fluorescent dye (e.g., fluorescein or fluorescent protein), a radionuclide or a biotin.

### Kit

In the seventeenth aspect, the present application also provides a kit, which comprises the nanobody or antigen-binding fragment thereof as described above or the polypeptide construct as described above or the conjugate of the sixteenth aspect as described above.

In certain embodiments, the kit comprises the conjugate of the sixteenth aspect as described above.

In certain embodiments, the kit comprises the nanobody or antigen-binding fragment thereof as described above or the polypeptide construct as described above, and a second antibody capable of specifically recognizing the nanobody or antigen-binding fragment thereof or the polypeptide construct; optionally, the second antibody further comprises a detectable label, such as an enzyme (e.g., horseradish peroxidase or alkaline phosphatase), a chemiluminescent reagent (e.g., an acridinium ester compound, luminol and derivative thereof, or ruthenium derivative), a fluorescent dye (e.g., fluorescein or fluorescent protein), a radionuclide or a biotin.

### Detection method

In an eighteenth aspect, the present application also provides a method for detecting the presence or level of MSLN in a sample, which comprises using the nanobody or antigen-binding fragment thereof as described above or the polypeptide construct as described above or the conjugate of the sixteenth aspect as described above. In certain embodiments, the method is used for therapeutic purposes, diagnostic purposes, or non-therapeutic non-diagnostic purposes.

In certain embodiments, the method is an immunological assay, such as a Western blot, an enzyme immunoassay (e.g., ELISA), a chemiluminescent immunoassay, a fluorescent immunoassay, or a radioimmunoassay.

In certain embodiments, the method comprises using the conjugate of the sixteenth aspect as described above.

In certain embodiments, the method comprises using the nanobody or antigen-binding fragment thereof as described above or the polypeptide construct as described above, and the method further comprises using a second nanobody bearing a detectable label (e.g., an enzyme (e.g., horseradish peroxidase or alkaline phosphatase), a chemiluminescent reagent (e.g., an acridinium ester compound, luminol and derivative thereof, or ruthenium derivative), a fluorescent dye (e.g., fluorescein or fluorescent protein), a radionuclide or a biotin) to detect the nanobody or antigen-binding fragment thereof or the polypeptide construct.

In certain embodiments, the method comprises: (1) contacting the sample with the nanobody or antigen-binding fragment thereof of the present application, the polypeptide construct of the present application, or the conjugate of the sixteenth aspect of the present application; (2) detecting the formation of an antigen-antibody immune complex or detecting the amount of the immune complex. The formation of the immune complex indicates the presence of MSLN or MSLN-expressing cells.

In certain embodiments, the method is used to detect whether a tumor is treatable by an anti-tumor therapy targeting MSLN.

### Use for preparing detection reagent

In the nineteenth aspect, the present application also provides a use of the nanobody or antigen-binding fragment thereof as described above or the polypeptide construct as described above or the conjugate of the sixteenth aspect as described above in the manufacture of a detection reagent for detecting the presence or level of MSLN in a sample or detecting whether a tumor is treatable by an anti-tumor therapy targeting MSLN.

In certain embodiments, the detection reagent detects the presence or level of MSLN in the sample and optionally detects whether a tumor is treatable by an anti-tumor therapy targeting MSLN by the method of the eighteenth aspect as described above.

In certain embodiments, the sample is a cell sample (e.g., a sample containing tumor cells) or a body fluid sample (e.g., blood) from a subject (e.g., a mammal, for example, a human).

### Definition of Terms

In the present application, unless otherwise stated, scientific and technical terms used herein have the meanings commonly understood by those skilled in the art. Moreover, the virology, biochemistry, and immunology laboratory procedures used herein are routine procedures widely used in the corresponding fields. Meanwhile, in order to better understand the present application, definitions and explanations of relevant terms are provided below.

When the terms "e.g.," "such as," "for example," "comprise," "include," or variations thereof are used herein, these terms will not be considered limiting terms and will instead be interpreted to mean "without limitation" or "not limited to."

Unless otherwise indicated herein or clearly contradicted by context, the terms "a" and "an" as well as "the" and similar referents in the context of describing the present application (especially in the context of the following claims) are to be construed to cover singular and plural.

As used herein, the term "camelid antibody" refers to an antibody against an antigen, which is generated by immunizing or challenging an animal of the family Camelidae (including Camel, Alpaca, and L.glama) with the antigen. It is known to those skilled in the art that among the antibodies produced by animals of the family Camelidae, there is a "camelid heavy-chain antibody (HCAb)" that lacks light chain. Such antibody only contains one variable domain of heavy chain of HCAb (VHH) and two conventional CH2 and CH3 regions, and the VHH region cloned and expressed separately has good structural stability and antigen-binding activity. VHH is currently known as the smallest unit to be able to bind a target antigen.

As used herein, the term "nanobody" has the meaning commonly understood by those skilled in the art and refers to an antibody fragment consisting of a single monomeric variable antibody domain (e.g., a single heavy chain variable region), which is usually derived from a variable region of a heavy chain antibody (e.g., a camelid or shark antibody). Typically, nanobody is composed of 4 framework regions and 3 complementarity-determining regions, with the structure of FR1-CDR1-FR2-CDR2-FR3-CDR3-FR4. Nanobodies can be truncated at the N- or C-terminus so that they contain only part of FR1 and/or FR4, or lack one or two of those framework regions, as long as they substantially retain antigen-binding ability and specificity. Nanobody is also called single-domain antibody (sdAb), and the two are used interchangeably.

As used herein, the term "antigen-binding fragment" of a nanobody refers to a polypeptide comprising a fragment of the nanobody that retains the ability to specifically bind the same antigen to which the nanobody binds, and/or competes with the nanobody for specific binding the antigen, which is also called "antigen-binding moiety". See generally, Fundamental Immunology, Ch. 7 (Paul, W., ed., 2nd ed., Raven Press, N.Y. (1989), which is incorporated herein by reference in its entirety for all purposes. The antigen-binding fragment of the antibody of the present application can be obtained by recombinant DNA technology or by enzymatic or chemical cleavage of the nanobody of the present application. In some embodiments, compared to the full-length nanobody, the "antigen-binding fragment" of the nanobody can be truncated at the N-terminus or C-terminus so that it contains only part of FR1 and/or FR4, or lacks one or two of those framework regions, as long as it substantially retains the antigen-binding ability and specificity.

An antigen-binding fragment of nanobody can be obtained from a given nanobody (e.g., the nanobody provided by the present application) using conventional techniques known to those skilled in the art (e.g., recombinant DNA technology or enzymatic or chemical fragmentation methods), and the antigen-binding fragment of nanobody can be screened for specificity in the same manner as for the intact nanobody.

In this document, when the term "nanobody" is mentioned, it includes not only an intact nanobody, but also antigen-binding fragment of the nanobody, unless the context clearly indicates otherwise.

As used herein, the term "complementarity-determining region" or "CDR" refers to the amino acid residues in a variable region of an antibody that are responsible for antigen binding. The nanobody contains three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given Nanobody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

As used herein, the term "framework region" or "FR" residues refers to those amino acid residues in antibody variable region other than the CDR residues as defined above.

As used herein, the term "Fc domain" or "Fc region" refers to a portion of a heavy chain constant region that contains CH2 and CH3 domains. Fc fragment of an antibody has many different functions but does not participate in antigen binding. "Effector functions" mediated by Fc region include Fc receptor binding; Clq binding and complement-dependent cytotoxicity (CDC); antibody-dependent cell-mediated cytotoxicity (ADCC); phagocytosis; down-regulation of cell surface receptors (e.g., B-cell receptors); and B-cell activation, etc. In some embodiments, the Fc region comprises a hinge, a CH2 domain, and a CH3 domain. When the Fc region comprises a hinge, the hinge mediates dimerization between two Fc-containing polypeptides. The Fc region can be of any antibody heavy chain constant region isotype, such as IgGl, IgG2, IgG3 or IgG4.

The Fc domain may include either a native Fc region or a variant Fc region. Native Fc region comprises an amino acid sequence that is consistent with the amino acid sequence of Fc region found in nature, for example, native human Fc region includes human IgG1 (non-A and A allotypes) Fc region with a native sequence; human IgG2 Fc region with a native sequence; human IgG3 Fc region with a native sequence; and human IgG4 Fc region with a native sequence, as well as naturally occurring variants thereof. The variant Fc region comprises an amino acid sequence that differs from the amino acid sequence of native Fc region due to at least one amino acid modification. In some embodiments, the variant Fc region may possess altered effector functions (e.g., Fc receptor binding, antibody glycosylation, number of cysteine residues, effector cell function, or complement function) compared to the native Fc region.

As used herein, the term "humanized antibody" refers to a non-human antibody that has been genetically engineered and of which amino acid sequence has been modified to increase sequence homology to that of a human antibody. Generally speaking, all or part of the CDR regions of a humanized antibody are derived from a non-human antibody (donor antibody), and all or part of the non-CDR regions (e.g., FR in variable region and/or constant region) are derived from a human immunoglobulin (receptor antibody). In certain embodiments, the CDR regions of the humanized antibody are derived from a non-human antibody (donor antibody), and all or part of the non-CDR regions (e.g., FR in variable region and/or constant region) are derived from a human immunoglobulin (receptor antibody). The humanized antibody generally retains the expected properties of the donor antibody, including but not limited to, antigen specificity, affinity, reactivity, etc. In the present application, the donor antibody may be a camelid antibody with desired properties (e.g., antigen specificity, affinity, reactivity, etc.). To prepare humanized antibodies, the CDR regions of the antibody from immunized animals can be inserted into human framework sequences using methods known in the art. In the context of nanobodies, humanized antibodies may refer to humanized VHHs, i.e., VHHs in which one or more framework regions have been substantially replaced by human framework regions. In some cases, certain framework regions (FRs) of human immunoglobulins are replaced by corresponding non-human residues. Additionally, a humanized VHH may contain residues that are not found in either the original VHH or the human framework sequence, but are included to further improve and optimize the performance of the VHH or VHH-containing polypeptide.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in a first amino acid sequence or nucleic acid sequence to best match a second amino acid or nucleic acid sequence). The amino acid residues or nucleotides at the corresponding amino acid positions or nucleotide positions are then compared. The molecules are identical when a position in the first sequence is occupied by the same amino acid residue or nucleotide as the corresponding position in the second sequence. The percent identity between two sequences is a function of the number of identical positions shared by the sequences (i.e., percent identity = number of identical overlapping positions/total number of positions × 100%). In certain embodiments, both sequences are of the same length.

Determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, which had been improved by Karlin and Altschul, 1993, Proc. Natl. Acad. Sci. U.S.A. 90:5873-5877. Such algorithms are integrated into the NBLAST and XBLAST programs of Altschul et al., 1990, J. Mol. Biol. 215:403.

As used herein, the term "specific binding" refers to a non-random binding reaction between two molecules, such as the binding reaction between an antibody and an antigen to which it is directed. The strength or affinity of a specific binding interaction can be expressed by the equilibrium dissociation constant (K_{D}) of the interaction. In the present application, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding property between two molecules can be determined using methods known in the art. One approach involves measuring the rate at which antigen binding site/antigen complex forms and dissociates. Both the "association rate constant" (kₐ or kₒₙ) and the "dissociation rate constant" (k_{dis} or k_{off}) can be calculated from the concentration and the actual rates of association and dissociation (see Malmqvist M, Nature, 1993, 361 :186-187). The ratio k_{dis}/kₒₙ is equal to the dissociation constant K_{D} (see Davies et al., Annual Rev Biochem, 1990; 59:439-473). K_{D}, kₒₙ and k_{dis} values can be measured by any valid method. In certain embodiments, the dissociation constant can be measured by surface plasmon resonance (SPR) with a Biacore instrument. Besides, bioluminescence interferometry or Kinexa can be used to measure the dissociation constant.

As used herein, a detectable label of the present application may be any substance detectable by fluorescent, spectroscopic, photochemical, biochemical, immunological, electrical, optical or chemical means. Such labels are well known in the art and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750)), luminescent substances (e.g., chemiluminescent substances, such as acridinium ester compounds, luminol and derivatives thereof, ruthenium derivatives such as ruthenium terpyridyl), magnetic beads (e.g., Dynabeads^{®}), calorimetric markers such as colloidal gold or colored glass or plastic (e.g., polystyrene, polypropylene, latex, etc.) beads, and biotin that binds to avidin (e.g., streptavidin) modified with the above label.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC) or P1-derived artificial chromosome (PAC); phages such as λ phage or M13 phage as well as animal viruses, etc. The animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of expression-controlling elements, including, but not limited to, promoter sequence, transcription initiation sequence, enhancer sequence, selection element, and reporter gene. In addition, the vector may also contain an origin of replication.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* insect cell such as *S2 Drosophila* cells or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, the conservative substitution can be introduced by standard techniques known in the art, such as site-directed mutagenesis and PCR-mediated mutagenesis. Conservative amino acid substitutions include those in which an amino acid residue is replaced with another amino acid residue having a similar side chain, for example, one that is physically or functionally similar to the corresponding amino acid residue (e.g., having similar size, shape, charge, chemical properties, including ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which are incorporated herein by reference).

The twenty conventional amino acids involved in this article are written in accordance with the conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning and are used interchangeably. And in the present application, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995) and includes, but is not limited to: pH adjuster, surfactant, adjuvant, ionic strength enhancer, diluent, agent for maintaining osmotic pressure, agent for delay absorption, preservative. For example, the pH adjusting agent includes, but is not limited to, phosphate buffer. The surfactant includes, but is not limited to, cationic, anionic or nonionic surfactant such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizer has the meaning generally understood by those skilled in the art, which can stabilize the desired activity of an active ingredient in medicine, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

As used herein, the term "prevention" refers to a method performed to prevent or delay the occurrence of a disease or condition or symptom in a subject. As used herein, the term "treatment" refers to a method performed to obtain a beneficial or desired clinical outcome. For the purposes of the present application, beneficial or desired clinical outcome includes, but is not limited to, alleviation of symptoms, reduction of the extent of disease, stabilization (i.e., no worsening) of the state of disease, delaying or slowing the progression of disease, amelioration or alleviation of the state of disease, and relief of symptom (whether partial or complete), whether detectable or undetectable. In addition, "treatment" may also refer to prolonging survival compared to expected survival if not receiving treatment.

As used herein, the term "subject" refers to a mammal, such as a human, a monkey, a mouse. In certain embodiments, the subject (e.g., human, monkey, mouse) has, or is at risk for, a disease associated with MSLN (e.g., a MSLN-positive tumor).

As used herein, the term "effective amount" refers to an amount sufficient to obtain, at least in part, the desired effect. For example, a prophylactically effective amount is an amount sufficient to prevent, arrest, or delay the onset of a disease (e.g., a MSLN-positive tumor); a therapeutically effective amount is an amount sufficient to cure or at least partially prevent an existing disease and complication thereof in a patient who is suffering the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner for drug administration, and other treatments administered concurrently, etc.

### Beneficial effects of the present application

The present application provides a nanobody with high binding activity to MSLN, which has cross-reactivity with human, monkey and/or mouse MSLN. In addition, the nanobody also has the characteristics of small molecular weight and good stability. Compared with traditional and common antibodies in drug development and diagnostic reagent development, it has good tissue infiltration, flexible administration, high humanization degree, easy transformation into recombinant protein and many other advantages.

Therefore, the nanobody of the present application can be used for a variety of purposes, including but not limited to inhibiting tumor growth and detecting MSLN. Furthermore, the fully humanized antibody of the present application can be safely administered to a human subject without eliciting an immunogenic response. Therefore, the antibody of the present application has significant clinical value.

The embodiments of the present application will be described in detail below with reference to the accompanying drawings and examples, but those skilled in the art will understand that the following drawings and examples are only used to illustrate the present application and do not limit the scope of the present application. Various objects and advantageous aspects of the present application will become apparent to those skilled in the art from the accompanying drawings and the following detailed description of preferred embodiments.

### Brief Description of the Drawings

Fig. 1 shows the detection results of the affinity of anti-MSLN nanobody to CHO-hMSLN cells.
Fig. 2 shows the detection results of the affinity of humanized anti-MSLN nanobody to CHO-hMSLN cells.
Fig. 3 shows the detection results of the affinity of humanized anti-MSLN nanobody to CHO-cyMSLN cells.
Fig. 4 shows the detection results of the affinity of humanized anti-MSLN nanobody to CHO-mMSLN cells.
Fig. 5 shows the detection results of the blocking activity of humanized anti-MSLN nanobody in blocking the binding of human MSLN to its ligand CA125.

### Sequence information

The sequences covered by the present application are described in the table below.

**Table 1: Sequence information**

| SEQ ID NO: | Sequence and description |
|---|---|
| 1 | Amino acid sequence of YE-17 CDR1 GTIFSRN |
| 2 | Amino acid sequence of YE-17 CDR2 |
| | ILNDGTT |
| 3 | Amino acid sequence of YE-17 CDR3 |
| | GYSDYRGTDY |
| 4 | Amino acid sequence of YE-17 VHH |
| | QVQLQESGGGLVQPGGSLRLSCATSGTIFSRNGMGWFRQAPGEQRELVARILND GTTMYADSVKGRFTISRDNAKNTVLLQMNSLKPEDTAVYYCGYSDYRGTDYW GQGTQVTVSS |
| 5 | Amino acid sequence of human IgG1-Fc fragment |
| | DKTHTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPI EKTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSP G |
| 6 | Amino acid sequence of HZ-YE-17-01 VHH |
| | QVQLVESGGGVVQPGGSLRLSCATSGTIFSRNGMGWFRQAPGEQRELVARILND GTTMYADSVKGRFTISRDNSKNTVLLQMNSLKPEDTAVYYCGYSDYRGTDYW GQGTQVTVSS |
| 7 | Amino acid sequence of HZ-YE-17-02 VHH |
| | QVQLVESGGGVVQPGGSLRLSCATSGTIFSRNGMGWFRQAPGKQRELVARILND GTTMYADSVKGRFTISRDNSKNTVYLQMNSLKPEDTAVYYCGYSDYRGTDYW GQGTQVTVSS |
| 8 | Amino acid sequence of HZ-YE-17-03 VHH |
| | QVQLVESGGGVVQPGGSLRLSCATSGTIFSRNGMGWFRQAPGKGLELVARILND GTTMYADSVKGRFTISRDNSKNTVYLQMNSLKPEDTAVYYCGYSDYRGTDYW GQGTQVTVSS |
| 9 | Amino acid sequence of HZ-YE-17-04 VHH |
| | QVQLVESGGGVVQPGGSLRLSCATSGTIFSRNGMGWFRQAPGKGLELVARILND GTTMYADSVKGRFTISRDNSKNTVYLQMNSLRAEDTAVYYCGYSDYRGTDYW GQGTQVTVSS |
| 10 | Amino acid sequence of human FR1 |
| | QVQLVESGGGVVQPGGSLRLSCATS |
| 11 | Amino acid sequence 1 of human FR2 |
| | MGWFRQAPGEQRELVAR |
| 12 | Amino acid sequence 2 of human FR2 |
| | MGWFRQAPGKQRELVAR |
| 13 | Amino acid sequence 3 of human FR2 |
| | MGWFRQAPGKGLELVAR |
| 14 | Amino acid sequence 1 of human FR3 |
| | MYADSVKGRFTISRDNSKNTVLLQMNSLKPEDTAVYYC |
| 15 | Amino acid sequence 2 of human FR3 |
| | MYADSVKGRFTISRDNSKNTVYLQMNSLKPEDTAVYYC |
| 16 | Amino acid sequence 3 of human FR3 |
| | MYADSVKGRFTISRDNSKNTVYLQMNSLRAEDTAVYYC |
| 17 | Amino acid sequence of human FR4 |
| | WGQGTQVTVSS |
| 18 | Amino acid sequence of amatuximab heavy chain |
| | QVQLQQSGPELEKPGASVKISCKASGYSFTGYTMNWVKQSHGKSLEWIGLITPY NGASSYNQKFRGKATLTVDKSSSTAYMDLLSLTSEDSAVYFCARGGYDGRGFD YWGSGTPVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEAL HNHYTQKSLSLSPGK |
| 19 | Amino acid sequence of amatuximab light chain |
| | DIELTQSPAIMSASPGEKVTMTCSASSSVSYMHWYQQKSGTSPKRWIYDTSKLA SGVPGRFSGSGSGNSYSLTISSVEAEDDATYYCQQWSKHPLTFGSGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |

### Specific Models for Carrying Out the present application

The present application will now be described with reference to the following examples which are intended to illustrate but not to limit the present application.

Unless otherwise specified, the molecular biology experimental methods and immunoassay methods used in the present application were performed by basically referring to the methods described in J. Sambrook et al., Molecular Cloning: Laboratory Manual, 2nd Edition, Cold Spring Harbor Laboratory Press, 1989, and F. M. Ausubel et al., Compiled Laboratory Guide to Molecular Biology, 3rd Edition, John Wiley & Sons, Inc., 1995; the use of restriction enzymes was in accordance with the conditions recommended by the product manufacturer. Those skilled in the art will appreciate that the examples describe the present application by way of example and are not intended to limit the scope of the present application as claimed.

### Example 1: Immunization and screening of anti-MSLN nanobody

After immunizing alpacas (Llama) with human MSLN (purchased from AcroBiosystems, Cat. No.: MSN-H522a), total RNA was extracted from alpaca peripheral lymphocytes and reverse transcribed to obtain cDNA. The PCR product of the cDNA was ligated to yeast display vector, and then electrotransformed into *Saccharomyces cerevisiae* (purchased from ATCC, Cat. No.: 208289) to construct an anti-MSLN nanobody library.

Human MSLN was labeled according to the product instructions of the biotin labeling kit (purchased from Thermo, Cat. No.: 90407). After the expanded anti-MSLN nanobody yeast library was labeled with biotin-labeled MSLN, magnetic beads were used to enrich the positively labeled yeast. After expansion of the yeast cells enriched by magnetic beads, 1:200 diluted anti-c-Myc antibody (purchased from Thermo, Cat. No.: MA1-980) and an appropriate amount of biotin-labeled MSLN were added for staining, and the yeast cells were washed with PBS, then added with 1:500 diluted goat-anti-mouse IgG (H+L) Alexa Fluor Plus 488 (purchased from Invitrogen, Cat. No.: A32723TR) and streptavidin APC Conjugate fluorescent antibody (purchased from Invitrogen, Cat. No.: SA1005), and incubated for 15 minutes. The cells were resuspended in PBS and sorted by a BD FACSAria II instrument to obtain yeast cells with higher binding ability to human MSLN.

The yeast cells with high binding ability to human MSLN obtained through enriching with magnetic beads and sorting by flow cytometry were cultured in an expansion medium at 30°C and 225 rpm overnight, and the yeast plasmid was extracted according to the operation of the yeast plasmid extraction kit (purchased from Tiangen (Cat. No. DP112). The plasmid was electrotransformed into Top10 competent cells (purchased from Tiangen, Cat. No.: CB104-02), coated on an ampicillin-resistant plate, and cultured at 37°C overnight. Single clones were picked for sequencing to obtain VHH (variable region) gene sequence, and the CDR region sequences were determined according to the IMGT numbering system. The sequence information of the obtained monoclonal nanobody YE-17 was shown in the table below.

**Table 2: Information of VHH and CDR sequences of nanobody**

| Clone No. | VHH (SEQ ID NO:) | CDR1 (SEQ ID NO:) | CDR2 (SEQ ID NO:) | CDR3 (SEQ ID NO:) |
|---|---|---|---|---|
| YE-17 | 4 | 1 | 2 | 3 |

### Example 2: Expression vector construction, protein expression and purification of anti-MSLN nanobody

The coding sequence of VHH of the screened anti-MSLN antibody YE-17 and the coding sequence of the human IgG1 Fc segment (SEQ ID NO: 5) were constructed through homologous recombination into a fusion protein expression sequence, in which the human IgG1 Fc segment was ligated to the C-terminus of the VHH. The ExpiCHO^{™} Expression System kit (purchased from Thermo, Cat. No.: A2910001) was used to transfer the prepared fusion protein expression plasmid at a medium amount into Expi-CHO cells (purchased from Thermo, Cat. No.: A2910002) according to the transfection method described in the product instructions. After 5 days of cell culture, the supernatant was collected and the target protein was purified using Protein A magnetic beads (purchased from GenScript, Cat. No.: L00723). The magnetic beads were resuspended in an appropriate volume (1 to 4 times the magnetic bead volume) of a binding buffer (PBS+0.1% Tween 20, pH 7.4), then added to a sample to be purified, and incubated at room temperature for 1 hour, shaking gently during the period. The sample was placed on a magnetic stand (purchased from Beaver), the supernatant was discarded, and the magnetic beads were washed three times with the binding buffer. Elution buffer (0.1M sodium citrate, pH 3.2) in a volume 3 to 5 times that of the magnetic beads was added, shaken at room temperature for 5 to 10 minutes, and placed back on the magnetic stand, and then the elution buffer was collected, transferred to a collection tube with neutralization buffer (1M Tris, pH 8.54) and mixed well to complete the preparation and obtain the purified anti-MSLN nanobody YE-17.

### Example 3: Protein-binding affinity detection of anti-MSLN nanobody

ForteBio affinity measurement was performed according to the existing method (Estep, P et al., High throughput solution based measurement of antibody-antigen affinity and epitope binning. MAbs, 2013.5(2):p.270-8). Briefly, a sensor was equilibrated offline in an analysis buffer for 30 min, then detected online for 60 s to establish a baseline, and the purified antibody obtained in Example 2 was loaded online onto AHQ sensor. Then the sensor was placed into 100 nM human MSLN (Uniprot ID: Q13421) for 5 minutes, and then the sensor was transferred to PBS for dissociation for 5 minutes. Kinetic analyze was performed using a 1:1 binding model. In addition, antibody amatuximab (CAS No.: 931402-35-6) was set in parallel as a control group. The amino acid sequences of heavy and light chains of the antibody amatuximab were shown in SEQ ID NOs: 18-19. The results were shown in Table 3.

**Table 3. Affinity of candidate molecule**

| Antibody No. | KD (M) | Kon (1/Ms) | Koff (1/s) |
|---|---|---|---|
| YE-17 | 1.88E-10 | 5.50E+05 | 1.04E-04 |
| amatuximab | 8.13E-10 | 1.53E+06 | 1.24E-03 |

### Example 4: Cell-binding affinity detection of anti-MSLN nanobody

CHO cells overexpressing human MSLN (Uniprot ID: Q13421), i.e., CHO-hMSLN cells, were prepared by transfection with pCHO1.0 vector (purchased from Invitrogen) comprising MSLN cDNA. The expanded cultured CHO-MSLN cells were adjusted to have a cell density of 2×10⁶ cells/ml, added to a 96-well flow plate, 100 µL/well, and centrifuged for later use. The purified anti-MSLN antibody YE-17 prepared in Example 2 was diluted with PBS, 3-fold dilution starting at 400 nM for a total of 12 serial dilutions. The above diluted samples were added to the above 96-well flow plate with cells, 100 µL/well, incubated at 4°C for 30 min, and washed twice with PBS. Goat F(ab')₂ Anti-Human IgG - Fc(PE) (purchased from Abcam, Cat. No.: ab98596) diluted 100-fold with PBS was added, 100 µL/well, incubated at 4°C for 30 min, and washed twice with PBS. PBS was added, 100 µl/well, to resuspend the cells, detection was performed on a CytoFlex (Bechman) flow cytometer, and the corresponding MFI (mean fluorescence intensity) value was calculated.

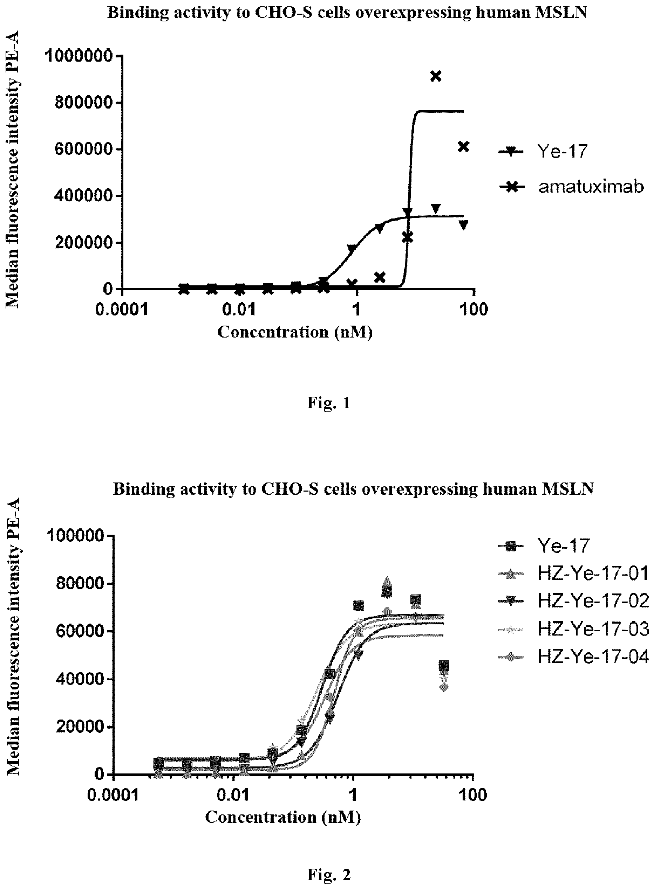

The experimental results were shown in Fig. 1 and Table 4. The experimental results showed that the anti-MSLN antibody YE-17 prepared in Example 2 had binding activity to CHO-hMSLN cells, and the binding activity was higher than that of the single-chain control antibody amatuximab from Morphotek.

**Table 4. EC₅₀ of anti-MSLN nanobody for binding to CHO-hMSLN cells**

| Antibody No. | EC₅₀ (nM) |
|---|---|
| YE-17 | 0.9647 |
| amatuximab | 1.531 |

### Example 5: Protein-binding affinity detection of humanized anti-MSLN nanobody

In this example, the antibody YE-17 was humanized. And the vector construction, expression and purification of humanized antibody were performed based on the sequence of the humanized antibody according to the methods described in Example 2. Finally, four strains of humanized antibodies were obtained based on YE-17, namely HZ-P-YE-17-01, HZ-P-YE-17-02, HZ-P-YE-17-03 and HZ-P-YE-17-04, and their VHH sequences were shown in SEQ ID NOs: 6-9, respectively.

The protein-binding affinity of the purified humanized antibodies was detected according to the method described in Example 3, and the results were shown in Table 5.

**Table 5: Detection results of protein-binding affinity of humanized anti-MSLN nanobodies**

| Antibody No. | KD (M) | Kon (1/Ms) | Koff (1/s) |
|---|---|---|---|
| YE-17 | 1.40E-09 | 4.21E+05 | 5.90E-04 |
| HZ-P-YE-17-01 | 8.07E-10 | 4.02E+05 | 3.25E-04 |
| HZ-P-YE-17-02 | 2.20E-09 | 4.03E+05 | 8.88E-04 |
| HZ-P-YE-17-03 | 1.91E-08 | 3.36E+05 | 6.40E-03 |
| HZ-P-YE-17-04 | 1.93E-08 | 3.29E+05 | 6.36E-03 |

### Example 6: Detection of binding ability of humanized anti-MSLN nanobodies to CHO-hMSLN and cross-reactivity thereof

The purified humanized antibodies were subjected to affinity detection against CHO-hMSLN cells according to the method described in Example 4.

The results were shown in Fig. 2 and Table 6. The results showed that the humanized anti-MSLN antibodies prepared in Example 5 all had binding activity to CHO-hMSLN cells.

**Table 6: EC₅₀ of humanized nanobodies for binding to CHO-hMSLN cells**

| No. | EC₅₀ (nM) |
|---|---|
| YE-17 | 0.305 |
| HZ-P-YE-17-01 | 0.4775 |
| HZ-P-YE-17-02 | 0.5515 |
| HZ-P-YE-17-03 | 0.2415 |
| HZ-P-YE-17-04 | 0.3399 |

In order to identify the affinity of the humanized antibodies to monkey MSLN (cyMSLN, Uniprot ID: F6Q1U7) at cellular level, the cell strain was constructed according to the method described in Example 4, and the affinity of the purified humanized antibodies to CHO-cyMSLN cells were detected.

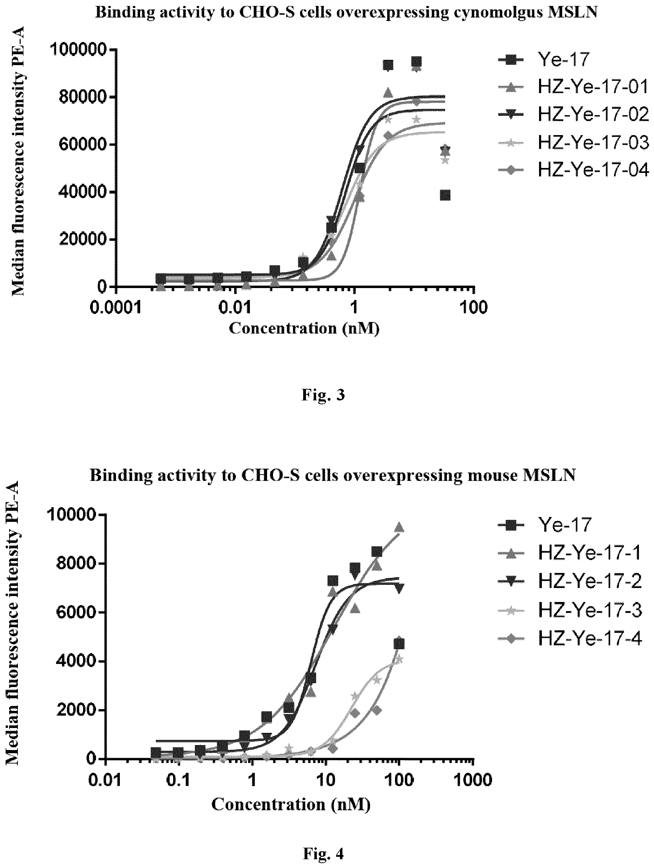

The results were shown in Fig. 3 and Table 7. The results showed that the antibody YE-17 and humanized anti-MSLN antibodies thereof all had binding activity to CHO-cyMSLN cells.

**Table 7: EC₅₀ of humanized nanobodies for binding to CHO-cyMSLN cells**

| No. | EC₅₀ (nM) |
|---|---|
| YE-17 | 0.7161 |
| HZ-P-YE-17-01 | 1.24 |
| HZ-P-YE-17-02 | 0.6298 |
| HZ-P-YE-17-03 | 0.7084 |
| HZ-P-YE-17-04 | 1.009 |

In order to identify the affinity of the antibodies to mouse MSLN (mMSLN, Uniprot ID: Q61468) at cellular level, the cell line was constructed according to the method described in Example 4, and the affinity of the purified humanized antibodies to CHO-mMSLN cells was detected, and the results were shown in Fig. 4 and Table 8.

**Table 8: EC₅₀ of humanized nanobodies for binding to CHO-mMSLN cells**

| No. | EC₅₀ (nM) |
|---|---|
| YE-17 | 6.312 |
| HZ-P-YE-17-01 | 12.99 |
| HZ-P-YE-17-02 | 7.293 |
| HZ-P-YE-17-03 | 22.35 |
| HZ-P-YE-17-04 | ∼ 249165 |

Combining the results in Tables 6 to 8 and Figs. 2 to 4, it was shown that the antibody YE-17 and its humanized antibodies had cross-binding activity to human and monkey MSLN cells, and that the antibody YE-17 and part of its humanized antibodies (e.g., HZ-P-YE-17-01, HZ-P-YE-17-02, HZ-P-YE-17-03) also had certain cross-binding activity to human, monkey and mouse MSLN cells.

### Example 7: Blocking binding of human MSLN to its ligand CA125 by Anti-MSLN humanized nanobodies

In order to identify the blocking activity of the antibodies in blocking the binding of human MSLN to its ligand CA125, experimental verification was carried out according to the following method: 1 µg/mL human MSLN was coated on a high-adsorption ELISA plate, and the coating was carried out at 4°C overnight; then the coating solution was discarded, and blocking was carried out with 5% BSA at room temperature for 2 hours; serially diluted humanized antibodies were added and incubated at room temperature for 1 hour; the plate was washed 3 times, then 1 µg/mL Biotin-CA125 (purchased from Aero, Cat. No.: CA5-H82F4) was added and incubated at room temperature for 1 hour; the plate was washed 3 times, then 1:10000 diluted SA-HRP (purchased from Abeam, Cat. No.: Ab7403) was added and incubated at room temperature for 30 minutes; the plate was washed 6 times, then 50 µl/mL TMB chromogenic solution was added and incubated for 3 to 5 minutes; 50 µl/mL stop solution was added; and A450 values were read on the machine.

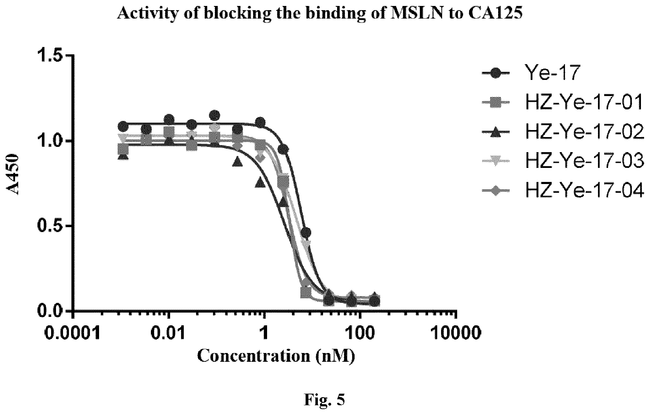

The results were shown in Fig. 5 and Table 9. The results showed that the antibody YE-17 and its humanized antibodies all could effectively block the binding of human MSLN to its ligand CA125, thereby inhibiting cancer invasion and metastasis.

**Table 9: EC₅₀ of humanized nanobodies in blocking human MSLN to bind its ligand CA125**

| No. | EC₅₀ (nM) |
|---|---|
| YE-17 | 5.958 |
| HZ-P-YE-17-01 | 3.342 |
| HZ-P-YE-17-02 | 2.627 |
| HZ-P-YE-17-03 | 4.753 |
| HZ-P-YE-17-04 | 3.234 |

Although the specific embodiments of the present application have been described in detail, those skilled in the art will understand that various modifications and changes can be made to the details based on all teachings that have been published, and these changes are within the protection scope of the present application. The entire application was given by the appended claims and any equivalents thereof.

## Claims

1. A nanobody or antigen-binding fragment thereof capable of specifically binding to MSLN, wherein the nanobody or antigen-binding fragment thereof comprises:
a CDR1 or variant thereof, a CDR2 or variant thereof, and a CDR3 or variant thereof contained in the variable region (VHH) as set forth in any one of SEQ ID NOs: 4 and 6-9;
wherein, the variant has a substitution, deletion or addition of one or several amino acids (e.g., a substitution, deletion or addition of 1, 2 or 3 amino acids) as compared to the sequence from which it is derived; preferably, the substitution is a conservative substitution;
preferably, the CDRs are defined according to the IMGT, Kabat or Chothia numbering system.

2. The nanobody or antigen-binding fragment thereof according to claim 1, wherein the nanobody or antigen-binding fragment thereof comprises:
a CDR1 having the sequence as set forth in SEQ ID NO: 1 or variant thereof, a CDR2 having the sequence as set forth in SEQ ID NO: 2 or variant thereof, and a CDR3 having the sequence as set forth in SEQ ID NO: 3 or variant thereof;
wherein, the variant has a substitution, deletion or addition of one or several amino acids (e.g., a substitution, deletion or addition of 1, 2 or 3 amino acids) as compared to the sequence from which it is derived; preferably, the substitution is a conservative substitution;
preferably, the nanobody or antigen-binding fragment thereof comprises: a CDR1 having the sequence as set forth in SEQ ID NO: 1, a CDR2 having the sequence as set forth in SEQ ID NO: 2, and a CDR3 having the sequence as set forth in SEQ ID NO: 3;
preferably, the CDRs are defined by the IMGT numbering system.

3. The nanobody or antigen-binding fragment thereof according to claim 1 or 2, which comprises an amino acid sequence selected from the following:
(i) the sequence as set forth in SEQ ID NO: 4;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 4; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 4;
preferably, the substitution is a conservative substitution.

4. The nanobody or antigen-binding fragment thereof according to any one of claims 1 to 3, wherein the nanobody or antigen-binding fragment thereof is humanized;
preferably, the nanobody or antigen-binding fragment thereof further comprises a heavy chain framework region of a human immunoglobulin (e.g., a heavy chain framework region contained in the amino acid sequence encoded by a human immunoglobulin heavy chain germline gene), and the heavy chain framework region optionally comprises one or more (e.g., 1, 2, 3, 4, 5, 6, 7, 8, 9 or 10) back mutations from human residues to camel residues.

5. The nanobody or antigen-binding fragment thereof according to any one of claims 1 to 4, wherein the antibody or antigen-binding fragment thereof comprises:
(i) an FR1 as set forth in SEQ ID NO: 10, or having a substitution, deletion or addition of one or several amino acids (e.g., a substitution, deletion or addition of 1, 2, 3, 4 or 5 amino acids) as compared to SEQ ID NO: 10, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to SEQ ID NO: 10;
(ii) an FR2 as set forth in any one of SEQ ID NOs: 11-13, or having a substitution, deletion or addition of one or several amino acids (e.g., a substitution, deletion or addition of 1, 2, 3, 4 or 5 amino acids) as compared to any one of SEQ ID NOs: 11-13, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to any one of SEQ ID NOs: 11-13;
(iii) an FR3 as set forth in any one of SEQ ID NOs: 14-16, or having a substitution, deletion or addition of one or several amino acids (e.g., a substitution, deletion or addition of 1, 2, 3, 4 or 5 amino acids) as compared to any one of SEQ ID NOs: 14-16, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to any one of SEQ ID NOs: 14-16;
and/or,
(iv) an FR4 as set forth in SEQ ID NO: 17, or having a substitution, deletion or addition of one or several amino acids (e.g., a substitution, deletion or addition of 1, 2, 3, 4 or 5 amino acids) as compared to SEQ ID NO: 17, or having a sequence identity of at least 80%, at least 85%, at least 90%, at least 91%, at least 92%, at least 93%, at least 94%, at least 95%, at least 96%, at least 97%, at least 98%, at least 99%, or 100% as compared to SEQ ID NO: 17.

6. The nanobody or antigen-binding fragment thereof according to any one of claims 1 to 5, wherein the nanobody or antigen-binding fragment thereof comprises an amino acid sequence selected from the following:
(i) the sequence as set forth in any one of SEQ ID NOs: 6-9;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 6-9; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 6-9;
preferably, the substitution is a conservative substitution.

7. A polypeptide construct capable of specifically binding to MSLN, which comprises the nanobody or antigen-binding fragment thereof according to any one of claims 1 to 6, and an immunoglobulin Fc domain;
preferably, the immunoglobulin Fc domain is ligated to the N-terminus and/or C-terminus (e.g., C-terminus) of the nanobody or antigen-binding fragment thereof, optionally via a peptide linker;
preferably, the immunoglobulin Fc domain is an IgG Fc domain (e.g., an IgG1 Fc domain);
preferably, the immunoglobulin Fc domain comprises the sequence as set forth in SEQ ID NO: 5, a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion or addition of 1, 2, 3, 4, or 5 amino acids) as compared thereto.

8. An isolated nucleic acid molecule, which encodes the nanobody or antigen-binding fragment thereof according to any one of claims 1 to 6 or the polypeptide construct according to claim 7.

9. A vector, which comprises the nucleic acid molecule according to claim 8; preferably, the vector is a cloning vector or an expression vector.

10. A host cell, which comprises the nucleic acid molecule according to claim 8 or the vector according to claim 9.

11. A method for preparing the nanobody or antigen-binding fragment thereof according to any one of claims 1 to 6 or the polypeptide construct according to claim 7, which comprises culturing the host cell according to claim 10 under a condition allowing protein expression, and recovering the nanobody or antigen-binding fragment thereof or the polypeptide construct from a culture of the cultured host cell.

12. A bispecific or multispecific antibody, which comprises the nanobody or antigen-binding fragment thereof according to any one of claims 1 to 6 or the polypeptide construct according to claim 7;
preferably, the bispecific or multispecific antibody specifically binds MSLN and additionally specifically binds one or more other targets;
preferably, the bispecific or multispecific antibody further comprises at least one second antibody having a second binding specificity for a second target.

13. A conjugate, which comprises the nanobody or antigen-binding fragment thereof according to any one of claims 1 to 6 or the polypeptide construct according to claim 7, and a therapeutic agent ligated to the nanobody or antigen-binding fragment thereof or the polypeptide construct;
preferably, the therapeutic agent is selected from the group consisting of anti-tumor drugs, such as cytotoxic agent, hormonal drug, biological response modifier, additional antibody or antigen-binding fragment thereof.

14. A chimeric antigen receptor, which comprises an antigen-binding domain, a spacer domain, a transmembrane domain and an intracellular signaling domain, wherein the antigen-binding domain comprises the nanobody or antigen-binding fragment thereof according to any one of claims 1 to 6;
preferably, the chimeric antigen receptor is expressed by an immune effector cell (e.g., a T cell).

15. An isolated nucleic acid molecule, which encodes the chimeric antigen receptor according to claim 14.

16. A vector, which comprises the isolated nucleic acid molecule according to claim 15; preferably, which is used to prepare a chimeric antigen receptor T cell.

17. A host cell, which comprises the isolated nucleic acid molecule according to claim 15 or the vector according to claim 16;
preferably, the host cell is an immune effector cell (e.g., a T cell or a NK cell);
preferably, the host cell is a chimeric antigen receptor T cell (CAR-T).

18. A pharmaceutical composition, which comprises the nanobody or antigen-binding fragment thereof according to any one of claims 1 to 6, the polypeptide construct according to claim 7, the isolated nucleic acid molecule according to claim 8, the vector according to claim 9, the host cell according to claim 10, the bispecific or multispecific antibody according to claim 12, the conjugate according to claim 13, the chimeric antigen receptor according to claim 14, the isolated nucleic acid molecule according to claim 15, the vector according to claim 16, or the host cell according to claim 17;
preferably, the pharmaceutical composition further comprises a pharmaceutically acceptable carrier and/or excipient;
preferably, the pharmaceutical composition comprises the nanobody or antigen-binding fragment thereof according to any one of claims 1 to 6, the polypeptide construct according to claim 7, the isolated nucleic acid molecule according to claim 8, the vector according to claim 9, or the host cell according to claim 10;
preferably, the pharmaceutical composition comprises the bispecific or multispecific antibody according to claim 12;
preferably, the pharmaceutical composition comprises the conjugate according to claim 13;
preferably, the pharmaceutical composition comprises the chimeric antigen receptor according to claim 14, the isolated nucleic acid molecule according to claim 15, the vector according to claim 16, or the host cell according to claim 17.

19. Use of the nanobody or antigen-binding fragment thereof according to any one of claims 1 to 6, the polypeptide construct according to claim 7, the isolated nucleic acid molecule according to claim 8, the vector according to claim 9, the host cell according to claim 10, the bispecific or multispecific antibody according to claim 12, the conjugate according to claim 13, the chimeric antigen receptor according to claim 14, the isolated nucleic acid molecule according to claim 15, the vector according to claim 16, the host cell according to claim 17, or the pharmaceutical composition according to claim 18 in the manufacture of a medicament for preventing and/or treating a tumor in a subject;
preferably, the tumor is a MSLN-positive tumor;
preferably, the tumor is selected from the group consisting of solid tumors, such as gastric cancer, lung cancer, ovarian cancer, esophageal cancer, pancreatic cancer, cervical cancer, mesothelioma or breast cancer;
preferably, the subject is a mammal, such as a human;
preferably, the nanobody or antigen-binding fragment thereof, polypeptide construct, isolated nucleic acid molecule, vector, host cell, bispecific or multispecific antibody, conjugate, chimeric antigen receptor, or pharmaceutical composition is used alone or in combination with an additional pharmaceutically active agent (e.g., an antineoplastic agent).

20. A method for preventing and/or treating a tumor in a subject, which comprises: administering to the subject in need thereof an effective amount of the nanobody or antigen-binding fragment thereof according to any one of claims 1 to 6, the polypeptide construct according to claim 7, the isolated nucleic acid molecule according to claim 8, the vector according to claim 9, the host cell according to claim 10, the bispecific or multispecific antibody according to claim 12, the conjugate according to claim 13, the chimeric antigen receptor according to claim 14, the isolated nucleic acid molecule according to claim 15, the vector according to claim 16, the host cell according to claim 17, or the pharmaceutical composition according to claim 18;
preferably, the tumor is a MSLN-positive tumor;
preferably, the tumor is selected from the group consisting of solid tumors, such as gastric cancer, lung cancer, ovarian cancer, esophageal cancer, pancreatic cancer, cervical cancer, mesothelioma or breast cancer;
preferably, the subject is a mammal, such as a human.

21. A conjugate, which comprises the nanobody or antigen-binding fragment thereof according to any one of claims 1 to 6 or the polypeptide construct according to claim 7, and a detectable label ligated to the nanobody or antigen-binding fragment thereof or the polypeptide construct; for example, the detectable label is an enzyme (e.g., horseradish peroxidase or alkaline phosphatase), a chemiluminescent reagent (e.g., an acridinium ester compound, luminol and derivative thereof, or ruthenium derivative), a fluorescent dye (e.g., fluorescein or fluorescent proteins), a radionuclides or a biotin.

22. A kit, which comprises the nanobody or antigen-binding fragment thereof according to any one of claims 1 to 6 or the polypeptide construct according to claim 7 or the conjugate according to claim 21;
preferably, the kit comprises the conjugate according to claim 21;
preferably, the kit comprises the nanobody or antigen-binding fragment thereof according to any one of claims 1 to 6 or the polypeptide construct according to claim 7, and a second antibody capable of specifically recognizing the nanobody or antigen-binding fragment thereof or the polypeptide construct; optionally, the second antibody further comprises a detectable label, such as enzyme (e.g., horseradish peroxidase or alkaline phosphatase), chemiluminescent reagent (e.g., an acridinium ester compound, luminol and derivative thereof, or ruthenium derivative), fluorescent dye (e.g., fluorescein or fluorescent protein), radionuclide or biotin.

23. A method for detecting the presence or level of MSLN in a sample, which comprises using the nanobody or antigen-binding fragment thereof according to any one of claims 1 to 6 or the polypeptide construct according to claim 7 or the conjugate according to claim 21;
preferably, the method is an immunological assay, such as Western blot, enzyme immunoassay (e.g., ELISA), chemiluminescent immunoassay, fluorescent immunoassay or radioimmunoassay;
preferably, the method comprises using the conjugate according to claim 21;
preferably, the method comprises using the nanobody or antigen-binding fragment thereof according to any one of claims 1 to 6 or the polypeptide construct according to claim 7, and the method further comprises using a second antibody bearing a detectable label (e.g., enzyme (e.g., horseradish peroxidase or alkaline phosphatase), chemiluminescent reagent (e.g., an acridinium ester compound, luminol and derivative thereof, or ruthenium derivative), fluorescent dye (e.g., fluorescein or fluorescent protein), radionuclide or biotin) to detect the nanobody or antigen-binding fragment thereof or the polypeptide construct.

24. Use of the nanobody or antigen-binding fragment thereof according to any one of claims 1 to 6 or the polypeptide construct according to claim 7 or the conjugate according to claim 21 in the manufacture of a detection reagent for detecting the presence or level of MSLN in a sample, or for detecting whether a tumor is treatable by an anti-tumor therapy targeting MSLN;
preferably, the detection reagent detects the presence or level of MSLN in the sample and optionally detects whether a tumor is treatable by an anti-tumor therapy targeting MSLN by the method according to claim 23;
preferably, the sample is a cell sample (e.g., a sample containing tumor cells) or a body fluid sample (e.g., blood) from a subject (e.g., a mammal, for example, a human).
